(19) Europäisches Patentamt
European Patent Office
Office européen des brevets

(11) Veröffentlichungsnummer: **0 270 963 B1**

(12) # EUROPÄISCHE PATENTSCHRIFT

(45) Veröffentlichungstag der Patentschrift: **07.08.91**

(51) Int. Cl.5: **C07D 261/08**, C07D 261/04

(21) Anmeldenummer: **87117652.5**

(22) Anmeldetag: **28.11.87**

Die Akte enthält technische Angaben, die nach dem Eingang der Anmeldung eingereicht wurden und die nicht in dieser Patentschrift enthalten sind.

(54) **Fluor enthaltende 5-Trihalogenmethyl-isoxazole und ein Verfahren zu deren Herstellung.**

(30) Priorität: **12.12.86 DE 3642453**

(43) Veröffentlichungstag der Anmeldung:
**15.06.88 Patentblatt 88/24**

(45) Bekanntmachung des Hinweises auf die Patenterteilung:
**07.08.91 Patentblatt 91/32**

(84) Benannte Vertragsstaaten:
**CH DE FR GB IT LI**

(56) Entgegenhaltungen:
**EP-A- 0 091 022**
**WO-A-83/00332**
**DE-A- 2 847 766**
**EP-A- 0 192 060**
**DE-A- 2 801 579**
**US-A- 4 000 150**

**CHEMICAL ABSTRACTS, Band 75, Nr. 9, 30. August 1971, Colombus, Ohio, USA; DEL'TSOVA, D.P. et al.: "Trifluoroacetonitrile N-Oxide", Seite 447, Spalte 2, Zusammenfassung-Nr. 63 699d**

(73) Patentinhaber: **BAYER AG**
**W-5090 Leverkusen 1 Bayerwerk(DE)**

(72) Erfinder: **Baasner, Bernd, Dr.**
**Hamberger Strasse 27d**
**W-5090 Leverkusen3(DE)**
Erfinder: **Klausener, Alexander, Dr.**
**Bendenstrasse 16**
**W-5190 Stolberg(DE)**
Erfinder: **Ooms, Pieter, Dr.**
**Dörperhofstrasse 16**
**W-4150 Krefeld(DE)**

**EP 0 270 963 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 5-Trihalogenmethyl-isoxazole der Formel (I)

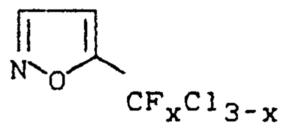 (I),

in der
x für 1, 2 oder 3 steht.

Die Formel (I) umfasst folgende Einzelverbindungen: 5-Trifluormethyl-isoxazol, 5-Difluorchlormethyl-isoxazol und 5-Fluordichlormethyl-isoxazol.

Die vorliegende Erfindung betrifft weiterhin ein Verfahren zur Herstellung von 5-Trihalogenmethyl-isoxazolen der Formel (I)

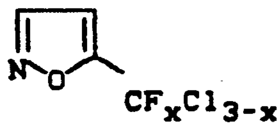 (I),

in der
x für 1, 2 oder 3 steht,
das dadurch gekennzeichnet ist, daß man 5-Trichlormethyl-isoxazol mit einem Fluorierungsmittel umsetzt. Als Fluorierungsmittel kommen beispielsweise Fluorwasserstoff und Antimontrifluorid infrage. Bevorzugt wird wasserfreier Fluorwasserstoff verwendet.

Es ist ausgesprochen überraschend, daß die neuen 5-Trihalogenmethyl-isoxazole der Formel (I) durch Umsetzung von 5-Trichlormethyl-isoxazol mit Fluorierungsmitteln wie Fluorwasserstoff und/oder der Lewis-Säure Antimontrifluorid, gegebenenfalls in Gegenwart weiterer Lewis-Säuren (z.B. Antimonchloride) zugänglich sind. Zum einen waren nämlich Umsetzungen des Isoxazol-Ringsystems mit Fluorwasserstoff und/oder Antimontrifluorid (z.B. Additionen) in Analogie zu anderen Umsetzungen von Isoxazolen mit Säuren (siehe z.B. A. Quilico Isoxazole and Related Compounds, pages 5-94 in The Chemistry of Heterocyclic Compounds (Ed. A. Weisberger), Band 17, New York-London, 1982) zu erwarten, zum anderen auch unter den angewendeten Fluorierungsbedingungen die Abspaltung der $CCl_3$-Gruppierung vom Isoxazol-Ring.

Das für das erfindungsgemäße Verfahren als Ausgangsprodukt benötigte 5-Trichlormethyl-isoxazol und seine Herstellung sind bekannt (siehe DE-OS 32 12 137). Für die erfindungsgemäße Herstellung von 5-Difluorchlormethyl- und 5-Trifluormethyl-isoxazol kann nicht nur von 5-Trichlormethyl-isoxazol ausgegangen werden, sondern auch von 5-Fluordichlormethyl-isoxazol oder Gemischen davon mit 5-Trichlormethyl-isoxazol. Für die erfindungsgemäße Herstellung von 5-Trifluormethyl-isoxazol kann auch von 5-Difluorchlormethyl-isoxazol oder Gemischen davon mit 5-Fluordichlormethyl-isoxazol und/oder 5-Trichlormethyl-isoxazol ausgegangen werden. Bei 5-Fluordichlormethyl-, 5-Difluorchlormethyl-isoxazol und diese enthaltenden Gemischen kann es sich um Zwischenstufen handeln, die bei der erfindungsgemäßen Herstellung von höher fluorierten Produkten (= 5-Difluorchlormethyl-oder 5-Trifluormethyl-isoxazol) durchlaufen werden.

Soweit als Fluorierungsmittel Fluorwasserstoff verwendet wird, wird dieser vorzugsweise im großen stöchiometrischen Überschuß eingesetzt, da der Überschuß dann als Lösungsmittel dienen kann. Beispielsweise kann man pro Mol Ausgangsmaterial (= 5-Trichlormethyl- und/oder 5-Fluordichlormethyl- und/oder 5-Difluorchlormethyl-isoxazol) 3 bis 1.500 Mol Fluorwasserstoff, insbesondere wasserfreien Fluorwasserstoff, einsetzen. Soweit als Fluorierungsmittel Antimontrifluorid verwendet wird, kann man beispielsweise die stöchiometrisch erforderliche Menge oder einen bis zu 30 mol-%igen Überschuß, im Falle der Herstellung von 5-Trifluormethyl-isoxazol auch einen bis zu 200 mol-%igen Überschuß einsetzen. Sonstige Fluorierungsmittel können beispielsweise in entsprechenden Mengen wie Antimontrifluorid eingesetzt werden.

Die erfindungsgemäße Fluorierung kann gegebenenfalls in Gegenwart üblicher Fluorierungskatalysatoren durchgeführt werden, wie sie z.B. in Houben-Weyl, Methoden der organischen Chemie, Georg-Thieme-Verlag, Stuttgart, Bd. 5/3, S. 124 ff. (1962) beschrieben sind. Besonders geeignet sind Antimonpentachlorid,

Gemische und/oder Umsetzungsprodukte von Antimontrichlorid und/oder Antimontrifluorid mit Chlor und/oder Fluorwasserstoff. Solche Umsetzungsprodukte können beispielsweise der Formel (II) entsprechen,

$SbCl_nF_{5-n}$ (II),

in der

n für eine ganze oder gebrochene Zahl von 1 bis 5 steht.

Vorzugsweise wird Antimonpentachlorid verwendet. Falls Fluorierungskatalysatoren eingesetzt werden, kann dies beispielsweise in Mengen von 0,005 bis 1 Mol pro Mol Ausgangsverbindung geschehen.

Die erfindungsgemäße Fluorierung wird im allgemeinen bei erhöhter Temperatur durchgeführt. Beispielsweise kann man bei 50 bis 250° C arbeiten. Der Druck kann beispielsweise im Bereich von 1 bis 50 bar (100 bis $5x10^3$ kPa) gewählt werden. Vorzugsweise arbeitet man bei der Verwendung von Fluorwasserstoff als Fluorierungsmittel diskontinuierlich in einem geschlossenen Gefäß unter dem sich bei der jeweiligen Reaktionstemperatur einstellenden Eigendruck oder drückt zusätzlich ein inertes Gas auf, z.B. Stickstoff, bis zu einem Gesamtdruck von bis zu 80 bar ($8x10^3$ kPa). Bei Verwendung von Antimontrifluorid als Fluorierungsmittel arbeitet man vorzugsweise bei Normaldruck oder Drucken bis zu 5 bar (500 kPa).

Geeignete Reaktionszeiten beim Einsatz von Fluorwasserstoff als Fluorierungsmittel sind beispielsweise solche im Bereich von 1 bis 24 Stunden.

Das erfindungsgemäße Verfahren kann auch kontinuierlich durchgeführt werden, wobei man die Fluorierung beispielsweise mit wasserfreiem Fluorwasserstoff in einem entsprechenden Reaktor in der Gasphase vornehmen kann. Dabei können beide Reaktionspartner (z.B. das 5-Trichlormethyl-isoxazol und der Fluorwasserstoff) in der Gasphase vorliegen und Drucke bis zu 50 bar ($5x10^3$ kPa) herrschen.

Die Gegenwart eines Lösungsmittels (Ausnahme überschüssiger Fluorwasserstoff) ist im allgemeinen nicht erforderlich.

Bei der Herstellung von 5-Fluordichlormethyl-, 5-Difluorchlormethyl- oder 5-Trifluormethyl-isoxazol sind jeweils geringfügig unterschiedliche Reaktionsbedingungen bevorzugt. Ganz allgemein fördern folgende Reaktionsbedingungen die Bildung höher fluorierte Produkte (siehe Formel (I) x = 2 oder 3):

- größere Überschüsse an Fluorierungsmittel, insbesondere bei Verwendung von Antimontrifluorid,
- Gegenwart von Fluorierungskatalysatoren, insbesondere in größeren Mengen,
- höhere Reaktionstemperaturen,
- höhere Reaktionsdrucke, insbesondere bei Verwendung von Fluorwasserstoff als Fluorierungsmittel und
- längere Reaktionszeiten.

Für die Herstellung von 5-Difluorchlormethyl- und 5-Trifluormethyl-isoxazol ist es jedoch nicht erforderlich, alle diese Reaktionsbedingungen in der Nähe der Obergrenze der weiter oben angegebenen Bereiche zu wählen. Im allgemeinen ist es dann ausreichend, eine oder zwei dieser Reaktionsbedingungen in der Nähe der Obergrenze der weiter oben angegebenen Bereiche und die sonstigen Reaktionsbedingungen beliebig zu wählen oder alle Reaktionsbedingungen etwa aus der Mitte oder der oberen Hälfte der weiter oben angegebenen Bereiche zu wählen.

Die erfindungsgemäße Herstellung von 5-Fluordichlormethyl-isoxazol (Formel (I), x = 1) erfolgt vorzugsweise mit 3 bis 500 Mol, besonders bevorzugt mit 5 bis 50 Mol Fluorwasserstoff pro Mol Ausgangsverbindung oder mit einem 5 bis 20 mol-%igen Überschuß an Antimontrifluorid, in Abwesenheit eines Fluorierungskatalysators, bei Temperaturen im Bereich von 50 bis 150° C, besonders bevorzugt 80 bis 130° C, bei Drucken von 1 bis 30 bar (100 bis $3x10^3$ kPa) besonders bevorzugt 1 bis 15 bar (100 bis $1{,}5x10^3$ kPa) und mit Reaktionszeiten von 2 bis 10, besonders bevorzugt 2 bis 5 Stunden.

Die erfindungsgemäße Herstellung von 5-Difluorchlorisoxazol (Formel (I), x = 2) erfolgt vorzugsweise mit 3 bis 1.000 Mol, besonders bevorzugt 8 bis 80 Mol Fluorwasserstoff pro Mol Ausgangsverbindung oder mit einem 10 bis 30 mol-5%igem Überschuß an Antimontrifluorid, unter Zusatz von 0,005 bis 0,2 Mol Fluorierungskatalysator, bei Temperaturen von 50 bis 200° C, besonders bevorzugt von 80 bis 160° C, bei Drucken von 2 bis 50 bar (200 bis $5x10^3$ kPa) besonders bevorzugt 10 bis 20 bar ($1x10^3$ bis $2x10^3$ kPa) (beim Einsatz von Fluorwasserstoff als Fluorierungsmittel) oder bei Drucken von 1 bis 5 bar (100 bis 500 kPa) besonders bevorzugt bei Normaldruck (beim Einsatz von Antimontrifluorid als Fluorierungsmittel) und mit Reaktionszeiten von 2 bis 15, besonders bevorzugt 3 bis 8 Stunden.

Die erfindungsgemäße Herstellung von 5-Trifluormethylisoxazol (Formel (I), x = 3) erfolgt vorzugsweise mit 10 bis 1.500 Mol, besonders bevorzugt 15 bis 200 Mol Fluorwasserstoff pro Mol Ausgangsverbindung oder mit einem 50 bis 200 mol-%igen Überschuß von Antimontrifluorid, unter Zusatz von 0,005 bis 1 Mol Fluorierungskatalysator, bei Temperaturen von 80 bis 250° C, besonders bevorzugt von 100 bis 190° C, bei der Verwendung von Fluorwasserstoff als Fluorierungsmittel bei Drucken von 15 bis 80 bar ($1{,}5x10^3$ bis

$8x10^3$ kPa) besonders bevorzugt 20 bis 35 bar ($2x10^3$ bis $3,5x10^3$ kPa) und bei der Verwendung von Antimontrifluorid also Fluorierungsmittel bei Drucken von 1 bis 5 bar (100 bis 500 kPa) besonders bevorzugt bei Normaldruck und mit Reaktionszeiten von 2 bis 20, besonders bevorzugt 5 bis 10 Stunden.

Beim Einsatz von Antimontrifluorid als Fluorierungsmittel können die zuvor angegebenen Reaktionszeiten auch kürzer gewählt werden und beispielsweise bei allen Herstellungsverfahren zwischen 0,1 und 10, besonders bevorzugt zwischen 0,5 und 5 Stunden betragen.

Die vorstehend für die Herstellung von 5-Difluorchlormethyl-isoxazol und 5-Trifluormethyl-isoxazol beschriebenen, bevorzugten und besonders bevorzugten Reaktionsbedingungen sind unabhängig davon, ob als Ausgangsprodukt 5-Trichlormethyl-isoxazol oder ein entsprechendes, bereits partiell fluoriertes Produkt eingesetzt wird.

Die Durchführung des erfindungsgemäßen Verfahrens kann beispielsweise so erfolgen, daß man 5-Trichlormethyl-isoxazol und das jeweilige Fluorierungsmittel, gegebenenfalls zusammen mit einem Fluorierungskatalysator, bei Raumtemperatur oder unter Kühlung (letzteres insbesondere beim Einsatz von Fluorwasserstoff als Fluorierungsmittel) zusammenbringt, gegebenenfalls mit Stickstoff einen Vordruck einstellt, danach auf Reaktionstemperatur aufheizt, nach Beendigung der Reaktion wieder abkühlt, bei Verwendung geschlossener Reaktionsgefäße den entstandenen Chlorwasserstoff entspannt und das Reaktionsgemisch aufarbeitet. Diese Aufarbeitung kann beispielsweise so erfolgen, daß man zunächst überschüssiges Fluorierungsmittel und gegebenenfalls vorhandenen Fluorierungskatalysator entfernt, z.B. durch Destillation und/oder Waschen mit Wasser und anschließend das so erhaltene Rohprodukt bei normalem oder vermindertem Druck fraktioniert. Man kann das Reaktionsgemisch aber auch auf andere Weise aufarbeiten, beispielsweise durch Zusatz von Wasser (gegebenenfalls erst nach dem Abdestillieren von Fluorwasserstoff), Abtrennen der organischen Phase, Waschen der wäßrigen Phase mit einem inerten organischen Lösungsmittel und Fraktionierung der vereinigten organischen Phasen bei normalem oder vermindertem Druck.

Die neuen 5-Trihalogenmethyl-isoxazole der Formel (I)

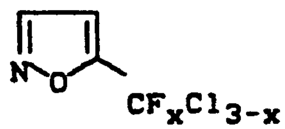

(I),

in der
x für 1, 2 oder 3 steht,
sind wertvolle Zwischenprodukte, aus denen 3,5-di-(halogenalkyl)-substituierte Pyrazole hergestellt werden können, die insektizide Wirkungen zeigen. Aus WO 83/00332 sind 5-halogenalkyl-substituierte Pyrazole bekannt, bei denen es sich um Herbizide handelt.

Die folgenden Beispiele erläutern die vorliegende Erfindung, ohne deren Umfang zu begrenzen.

Beispiele
1 bar = $1x10^5$ Pascal

Beispiel 1 (Synthese von 5-Fluordichlormethyl-isoxazol aus 5-Trichlormethyl-isoxazol)

Zu 120 ml wasserfreiem Fluorwasserstoff, die in einem Rührautoklaven aus Stahl vorgelegt waren, wurden bei O bis +3 °C in 60 Minuten 93,25 g (0,5 Mol) 5-Trichlormethyl-isoxazol zugetropft. Nach Aufdrücken von 5 bar Stickstoff wurde auf 115 °C aufgeheizt, wobei sich ein Druck von 12 bar einstellte und 3,5 Stunden bei dieser Temperatur belassen. Nach dem Abkühlen wurde entspannt, der Überschuß an Fluorwasserstoff bei 150 mbar und anschließend das Rohprodukt bei 20 mbar aus dem Reaktionsgefäß abdestilliert. Das so erhaltene Rohprodukt wurde zweimal mit je 200 ml Wasser gewaschen, die organische Phase mit Magnesiumsulfat getrocknet und bei 20 mbar destilliert. Es wurden 57 g (= 67 % der Theorie) an 5-Fluordichlormethyl-isoxazol (Formel (I), x = 1) mit einem Siedepunkt von 52 bis 54 °C bei 20 mbar und einem Brechungsindex $n_D^{20}$ von 1,4550 erhalten.

Beispiel 2 (Synthese von 5-Difluorchlormethyl-isoxazol aus 5-Trichlormethyl-isoxazol)

Zu 180 ml wasserfreiem Fluorwasserstoff und 0,5 ml Antimonpentachlorid, die in einem Rührautoklaven aus Stahl vorgelegt wurden, wurden bei -5 bis 0 °C in 40 Minuten 93,25 g (0,5 Mol) 5-Trichlormethyl-isoxazol zugetropft. Nach Aufdrücken von 18 bar Stickstoff wurde auf 150 °C aufgeheizt und 4 Stunden bei dieser Temperatur reagieren gelassen. Nach dem Abkühlen wurde der Überschuß an Fluorwasserstoff bei 150 mbar abdestilliert und das Rohprodukt dreimal mit je 200 Wasser gewaschen. Die organische Phase wurde nach dem Trocknen mit Magnesiumsulfat bei Normaldruck destilliert. Es wurden 43,2 g (=56,5 % der Theorie) an 5-Difluorchlormethyl-isoxazol (Formel (I), x = 2) mit einem Siedepunkt von 92 bis 93 °C und einem Brechungsindex $n_D^{20}$ von 1,4033 erhalten.

<u>Beispiel 3</u> (Synthese von 5-Trifluormethyl-isoxazol aus 5-Trichlormethyl-isoxazol)

56 g (0,3 Mol) 5-Trichlormethyl-isoxazol, 80 g Antimontrifluorid und 1 ml Antimonpentachlorid wurden bei Raumtemperatur zusammengefügt, dann auf eine Innentemperatur von 140 °C aufgeheizt und bei dieser Temperatur 90 Minuten bei Rückfluß reagieren gelassen. Anschließend wurden die flüchtigen Anteile bei Normaldruck abdestilliert (70 bis 120 °C Kopftemperatur) und das erhaltene Rohprodukt über eine 10 cm-Füllkörperkolonne redestilliert. Es wurden 26,8 g (=65,2 % der Theorie) an 5-Trifluormethylisoxazol (Formel (I), x = 3) mit einem Siedepunkt von 79 bis 80 °C bei Normaldruck und einem Brechungsindex $n_D^{20}$ von 1,3493 erhalten.

Bei 81 bis 110 °C wurden 16 g einer weiteren Fraktion erhalten, die nach gaschromatografischer Analyse zu 68,8 Gew.-% aus 5-Difluorchlormethyl-isoxazol und zu 30,3 Gew.-% aus 5-Trifluormethyl-isoxazol bestand. Diese Fraktion wurde bei nachfolgende, analogen Ansätzen zusammen mit 5-Trichlormethyl-isoxazol wieder eingesetzt.

<u>Beispiel 4</u> (Synthese von 5-Trifluormethyl-isoxazol aus 5-Fluordichlormethyl-isoxazol)

Zu 60 ml wasserfreiem Fluorwasserstoff und 0,5 ml Antimonpentachlorid, die in einem Rührautoklaven aus Stahl vorgelegt wurden, wurden bei -5 bis 0 °C in 20 Min. 70 g (0,1 Mol) 5-Fluordichlormethyl-isoxazol zugetropft. Nach Aufdrücken von 25 bar Stickstoff wurde auf 180 °C aufgeheizt und 8 Stunden bei dieser Temperatur reagieren gelassen. Nach dem Abkühlen wurde der Überschuß an Fluorwasserstoff bei 150 mbar abdestilliert und das Rohprodukt zweimal mit je 50 ml Wasser gewaschen. Die organische Phase wurde nach dem Trocknen mit Magnesiumsulfat bei Normaldruck destilliert. Es wurden 5,7 g (= 42 % d.Th.) an 5-Trifluormethyl-isoxazol (Formel (I), x = 3) mit einem Siedepunkt von 78 bis 80 °C und einem Brechungsindex $n_D^{20}$ von 1,3498 erhalten.

<u>Beispiel 5</u> (Synthese von 5-Trifluormethyl-isoxazol aus 5-Difluorchlormethyl-isoxazol)

30,7 g (0,2 Mol) 5-Difluorchlormethyl-isoxazol, 70 g Antimontrifluorid und 1 ml Antimonpentachlorid wurden bei Raumtemperatur zusammengefügt, dann auf eine Temperatur von etwa 140 °C (Badtemperatur) aufgeheizt und 90 Min. bei Rückfluß reagieren gelassen. Anschließend wurden die flüchtigen Anteile bei Normaldruck abdestilliert (Kopftemperatur 65 bis 105 °C) und das erhaltene Rohprodukt über eine 10 cm-Füllkörperkolonne redestilliert. Es wurden 19,75 g (= 72,1 % d.Th.) an 5-Trifluormethyl-isoxazol (Formel (I), x = 3) mit einem Siedepunkt von 79 bis 80 °C bei Normaldruck und einem Brechungsindex $n_D^{20}$ von 1,3495 erhalten.

<u>Beispiel 6</u> (Charakterisierung der gemäß den Beispielen 1 bis 3 hergestellten Produkte

Von den gemäß den Beispielen 1 bis 3 hergestellten Produkten wurden jeweils das $^1$H-NMR-Spektrum, das $^{19}$F-NMR-Spektrum un das Massenspektrum aufgenommen.

Bei allen NMR-Messungen diente $CDCl_3$ (= Deuterochloroform) als Lösungsmittel. Für die $^1$H-NMR-Messungen wurde TMS (= Tetramethylsilan) als interner, für die $^{19}$F-NMR-Messungen $CF_3COOH$ (= Trifluoressigsäure) als externer Standard verwendet.

Die NMR-Messungen erfolgten auf einem Bruker WP 80 FT-Spektrometer mit einem $^1$H/$^{19}$F-Dual Probenkopf bei einer Meßfrequenz von 80 MHz (für $^1$H) und 75,39 MHz (für $^{19}$F).

Die massenspektrografischen Daten wurden auf einem Massenspektrometer des Typs Finnigan MAT 112 (EI-mode, 70 eV) ermittelt.

In der nachfolgenden Tabelle sind die NMR-Daten und die Hauptpeaks der Massenspektren aufgeführt.

Tabelle 1

| Verbindung | $^1H$-NMR $\delta$[ppm] | $^{19}F$-NMR | Massespektrum |
|---|---|---|---|
| 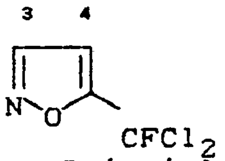 aus Beispiel 1 | 8,35 (4-H)<br>6,68 (3-H) | -27,47 | m/e 169/171 ($M^+$)<br>134/136 ($M^+$-Cl), base-peak |
| 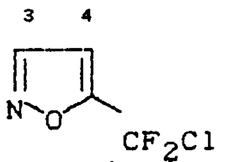 aus Beispiel 2 | 8,32 (4-H)<br>6,68 (3-H) | -27,40 | m/e 153/155 ($M^+$)<br>134/136 ($M^+$-F)<br>118 ($M^+$-Cl), base peak |
| 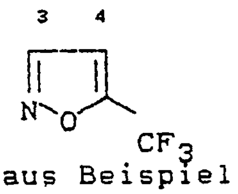 aus Beispiel 3 | 8,38 (4-H)<br>6,76 (3-H) | -14,39 | m/e 137 ($M^+$), base-peak<br>118 ($M^+$-F) |

Alle $^1H$- und $^{19}F$-NMR-Signale erscheinen als schwach aufgespaltete Singuletts, die Signale des jeweils in der Position 3 gebundenen Protons sind durch long-range-Kopplungen mit Fluor zusätzlich aufgespalten. Analog zeigen alle Fluorresonanzsignale Feinaufspaltung.

Beispiel 7 (Umsetzung von 5-Trifluormethylisoxazol zu einem Insektizid und dessen Wirkung)

Es wurden, 0,92 g (0,04 Mol) Natrium in 25 ml Ethanol gelöst, bei 0°C 5,48 g (0,04 Mol) 5-Trifluormethylisoxazol zugegeben und 15 Minuten bei Raumtemperatur gerührt. Zu diesem Gemisch wurde unter Kühlung 16,16 g (0,04 Mol) N-(2,6-Dichlor-4-trifluormethylphenyl)-trifluoracethydrazid-bromid zugetropft und noch 10 Stunden bei 25°C nachgerührt. Nach Abfiltrieren des ausgefallenen Natriumbromids wurde das Filtrat eingeengt und säulenchromatographisch aufgetrennt.

Es wurden 1,65 g (9,3 % der Theorie) 4-Cyan-3,5-di(trifluormethyl)-1-(2,6-dichlor-4-trifluormethylphenyl)-pyrazol vom Schmelzpunkt 88 - 92°C erhalten (im folgenden als Wirkstoff bezeichnet).

$LD_{100}$-Test

2 Gewichtsteile Wirkstoff wurden in 1000 volumenteilen Aceton aufgenommen. 2,5 ml der Wirkstofflösung wurden in eine Petrischale pipettiert. Auf dem Boden der Petrischale befand sich ein Filterpapier mit einem Durchmesser von etwa 9,5 cm. Die Petrischale blieb so lange offen stehen, bis das Lösungsmittel vollständig verdunstet war. Anschließend wurden 20 Tiere der Art Blattella germanica in die Petrischale gegeben und mit einem Glasdeckel bedeckt.

Der Zustand der Testtiere wurde 3 Tage nach Ansetzen der Versuche kontrolliert. Dieser Test zeigte eine überlegene Wirkung des Wirkstoffs gegenüber dem Stand der Technik.

$LT_{100}$-Test für Dipteren

2 Gewichtsteile Wirkstoff wurden in 1000 Volumenteilen Aceton aufgenommen. 2,5 ml der Wirkstofflösung wurden in eine Petrischale pipettiert. Auf dem Boden der Petrischale befand sich ein Filterpapier mit einem Druchmesser von etwa 9,5 cm. Die Petrischale blieb so lange offen stehen, bis das Lösungsmittel vollständig verdunstet war. Anschließend wurden 20 Tiere der Art Musca domestica (resistent) in die Petrischale gegeben und mit einem Glasdeckel bedeckt.

Der Zustand der Testtiere wurde laufend kontrolliert. Es wurde diejenige Zeit ermittelt, welche für einen 100 %igen knock down-Effekt notwendig ist. Dieser Test zeigte eine überlegene Wirkung des Wirkstoffs gegenüber dem Stand der Technik.

## Patentansprüche

1. 5-Trihalogenmethyl-isoxazole der Formel

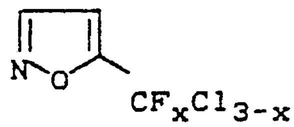

in der
x für 1, 2 oder 3 steht.

2. 5-Trifluormethyl-isoxazol, 5-Difluorchlormethyl-isoxazol und 5-Fluordichlormethyl-isoxazol.

3. Verfahren zur Herstellung von 5-Trihalogenmethyl-isoxazolen der Formel

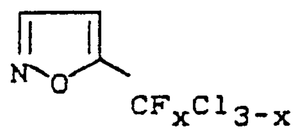

in der
x für 1, 2 oder 3 steht,
dadurch gekennzeichnet, daß man 5-Trichlormethyl-isoxazol mit Fluorwasserstoff oder Antimontrifluorid bei 50 bis 250 °C und 1 bis 80 bar (100 bis $8x10^3$ kPa) umsetzt.

4. Verfahren nach Anspruch 3, dadurch gekennzeichnet, daß man 3 bis 1.500 Mole Fluorwasserstoff pro Mal Ausgangsverbindung einsetzt.

5. Verfahren nach Anspruch 3, dadurch gekennzeichnet, daß man Antimontrifluorid zwischen der stöchiometrisch erforderlichen Menge und einem bis zu 30 mol-%igen Überschuß, im Falle der Herstellung von 5-Trifluormethyl-isoxazol zwischen der stöchiometrisch erforderlichen Menge und einem bis zu 200 mol-%igen Überschuß einsetzt.

6. Verfahren nach Ansprüchen 3 bis 5, dadurch gekennzeichnet, daß man Fluorierungskatalysatoren einsetzt.

7. Verfahren nach Ansprüchen 3 bis 6, dadurch gekennzeichnet, daß man bei der Verwendung von Fluorwasserstoff als Fluorierungsmittel Reaktionszeiten im Bereich von 1 bis 24 Stunden anwendet.

8. Verfahren nach Ansprüchen 4 bis 6, dadurch gekennzeichnet, daß man bei der Verwendung von Antimontrifluorid als Fluorierungsmittel Reaktionszeiten im Bereich von 0,1 bis 10 Stunden anwendet.

## Claims

1. 5-Trihalogenomethyl-isoxazoles of the formula

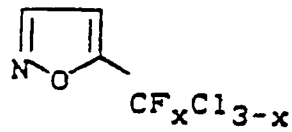

in which
x represents 1, 2 or 3.

2. 5-Trifluoromethyl-isoxazole, 5-difluorochloromethyl-isoxazole and 5-fluorodichloromethyl-isoxazole.

3. Process for the preparation of 5-trihalogenomethyl-isoxazoles of the formula

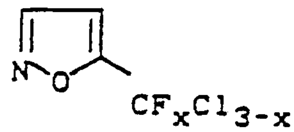

in which
x represents 1, 2 or 3,
characterised in that 5-trichloromethyl-isoxazole is reacted with hydrogen fluoride or antimony trifluoride at 50 to 250 °C and under 1 to 80 bar (100 to 8 x $10^3$ kPa).

4. Process according to Claim 3, characterised in that 3 to 1,500 moles of hydrogen fluoride are used per mole of starting compound.

5. Process according to Claim 3, characterised in that antimony trifluoride is used in an amount between the stoichiometric amount and an excess of up to 30 mol % and in the case of the preparation of 5-trifluoromethyl-isoxazole, between the stoichiometric amount and an excess of up to 200 mol %.

6. Process according to Claims 3 to 5, characterised in that fluorination catalysts are used.

7. Process according to Claims 3 to 7, characterised in that, when hydrogen fluoride is used as the fluorinating agent, reaction times in the range from 1 to 24 hours are used.

8. Process according to Claims 4 to 6, characterised in that, when antimony trifluoride is used as the fluorinating agent, reaction times in the range from 0.1 to 10 hours are used.

**Revendications**

1. 5-trihalogénométhylisoxazoles de formule

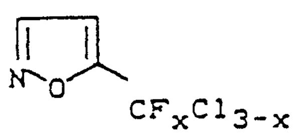

dans laquelle
x est égal à 1, 2 ou 3.

2. Le 5-trifluorométhylisoxazole, le 5-difluorochlorométhylisoxazole et le 5-fluorodichlorométhylisoxazole.

3. Procédé de préparation des 5-trihalogénométhylisoxazoles de formule

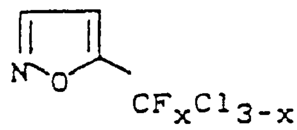

dans laquelle
x est égal à 1, 2 ou 3,
caractérisé en ce que l'on fait réagir le 5-trichlorométhylisoxazole avec le fluorure d'hydrogène ou le trifluorure d'antimoine à des températures de 50 à 250 °C sous des pressions de 1 à 80 bars (100 à 8 x $10^3$ kPa).

4. Procédé selon la revendication 3, caractérisé en ce que l'on met en oeuvre de 3 à 1 500 mol de fluorure d'hydrogène par mole du composé de départ.

5. Procédé selon la revendication 3, caractérisé en ce que l'on utilise le trifluorure d'antimoine en quantité allant de la quantité théorique jusqu'à un excès de 30 mol % et, dans le cas de la préparation du 5-trifluorométhylisoxazole, en quantité allant de la quantité théorique jusqu'à un excès de 200 mol %.

6. Procédé selon les revendications 3 à 5, caractérisé en ce que l'on utilise des catalyseurs de fluoration.

7. Procédé selon les revendications 3 à 6, caractérisé en ce que l'on observe des durées de réaction de 1 à 24 h lorsqu'on utilise le fluorure d'hydrogène comme agent fluorant.

8. Procédé selon les revendications 4 à 6, caractérisé en ce que l'on observe des durées de réaction de 0,1 à 10 h lorsqu'on utilise le trifluorure d'antimoine en tant qu'agent fluorant.